# EUROPEAN PATENT SPECIFICATION

(11) **EP 2 233 105 B1**
(45) Date of publication and mention of the grant of the patent: **10.10.2012**
(21) Application number: 10162129.0
(22) Date of filing: 01.07.2005
(51) Int. Cl.: A61C 5/06, A61C 9/00, A61M 39/10, B05C 17/005

(54) **Delivery system for dental materials**
System zur Zuführung von dentalen Werkstoffen
Système de distribution de matériaux dentaires

(43) Date of publication of application: 29.09.2010
(62) Divisional of application: 05014393.2
(73) Proprietor: 3M Innovative Properties Company, Saint Paul, MN 55133-3427 (US)
(72) Inventor: Peuker, Marc, 86938, Schondorf (DE); Bertl, Mathias, 82409, Wildsteig (DE); Peez, Robert, 86899, Landsberg (DE); Guggenmos, Sebastian, 82380, Peissenberg (DE); Müller, Peter, 82211, Herrsching (DE)
(74) Representative: Hohmann, Arno

(56) References cited:
- EP-A- 0 919 206
- EP-A1- 1 543 795
- DE-A1- 19 961 485
- US-A- 5 816 804

## Description

### Field of the invention

The present invention relates to a delivery system for dental materials, particularly for processing (such as storage, mixing and dispensing) of dental materials. In more particular, the present invention relates to a delivery system according to the preamble of claim 1.

### Background of the invention

Mixing capsules which are filled with the components in separate chambers by the manufacturer are used to produce mixtures of two or more components. The components are brought into communication with one another by the user, for example by destroying a wall separating the chambers, and are afterwards mixed, for example by shaking the capsule in a shaker unit.

Mixing capsules for the production of dental materials which are often mixed from a pulverulent component and a liquid component are known in the dental sector. The completely mixed substance is then dispensed directly onto the working area, for example into a tooth cavity, through a dispensing spout formed integrally on the mixing capsule.

EP-A-1 543 795 discloses a dental material application system comprising a multiple-use dental material dispensing cartridge and a single-use dispensing tip. The single-use dispensing tip is adapted for engagement with a dispensing tube of the multiple-use cartridge. However this engagement is releasable.

DE-U-90 03 983 describes a device for filling root channels with cement. The device comprises a cylindrical chamber with a displaceable piston, and a pivotable tubular element for dispensing material from the chamber. A flexible cannula is permanently affixed to the tip of the tubular element during manufacture of the device. Thus, the device can only be used with the cannula but not merely with the tubular element.

DE-A-199 61 485 describes a container for dispensing dental material. The device comprises a cylindrical chamber with a displaceable piston, and a pivotable tubular element for dispensing material from the chamber. A cannula is releasably connectable to the tubular element.

Other prior art relates to containers with dispensing tips with conical shapes which can be cut off to adapt the tip from a smaller to a larger diameter.

Further reference is made to US-A-6 135 771, DE-A-197 00 480, DE-A-19 39 315, and US 2002/098462.

Since there are plenty of cements on the market that can be used for a variety of applications, there are delivery systems available that cover the majority of them. However, for special treatments, such as post cementation, special separate delivery systems are provided. The provision of individual delivery systems for different treatments increases manufacturing costs.

### Summary of the invention

The present invention provides an improved delivery system for dental materials that provides flexibility in that it allows the use of the same capsule design for different dental treatments with different dental materials.

According to the present invention, a delivery system for dental materials is provided. The delivery system for dental materials comprises a capsule having a capsule body member and a cannula for dispensing material from the capsule body member, and an extension element, wherein the cannula and the extension element are adapted for a user-activatable, unreleasable engagement with each other.

The term "user-activatable" according to the present invention means that the extension element of the delivery system is not preassembled to the cannula of the capsule but is activatable, i.e., connectable to the cannula, by the user immediately prior to the use of the delivery system.

The term "unreleasable engagement" according to the present invention means that the extension element once connected to or engaged with the cannula by the user immediately prior to the use of the delivery system is permanently affixed to the cannula and cannot be removed again from the cannula, or can only be removed using strong forces.

The extension element for the cannula comprises a tubular back end portion and a tip portion. A tubular back end portion has a first average inner diameter, and the tip portion has a second average inner diameter, whereas the second average inner diameter is smaller than the first average inner diameter. The term "average diameter" encompasses constant diameters but also conical configurations having a diameter that decreases (or increases) along the length of the respective portion. Preferably, the tip portion is tapered towards its front end so that the tip or front end of the extension element is the smallest part of the extension element.

In order to provide a tight fit when the extension element and the cannula are connected with each other, the inner shape of the extension element corresponds to the outer shape of the cannula.

It is also preferred that the back end portion of the extension element comprises at least one aperture extending from the interior surface of the back end portion to its outer surface. Preferably, the axis of the aperture is perpendicular to the longitudinal axis of the extension element.

However, according to the present invention, the tubular back end portion is made from an elastic material. The elasticity of the tubular portion is such that the application of a pull force to it causes it to stretch and constrict, thereby increasing the frictional resistance between the inner surface of the tubular portion and the exterior surface of the cannula (when the cannula is inserted in the tubular portion). In this embodiment, retention elements like those described above in connection with the examples may also be provided.

Preferably, the elastic, tubular back end portion comprises at least one aperture extending from the interior surface of the tubular back end portion to its outer surface. More preferably, the aperture is provided in the form of a hole or longitudinal slot extending along the longitudinal axis of the extension element. The aperture ensures that dental material does not flow between the inner surface of the tubular portion and the exterior surface of the cannula during application of the dental material from the delivery system. If the frictional seal between the tubular back end portion of the extension element and the outer surface of the cannula is not adequate to essentially exclude dental material from flowing between the cannula and the tubular portion, the dental material may escape through the aperture rather than remaining between the cannula and tubular portion to adversely impact the frictional seal. This helps to prevent the extension element from coming off of the cannula during delivery of dental material from the capsule.

According to an aspect of the present invention, a kit for delivery of dental materials is provided. The kit according to the present invention comprises a delivery system according to claim 1 and an orally implantable post, e.g. a fibre post. Preferably a kit consists of a plurality of capsules and a plurality of extension elements, and, optionally, a plurality of posts, wherein the posts can be of different types, sizes, and materials, etc.

A preferred application for the delivery system according to the present invention is cementation of orally implantable posts. For post cementation, the delivery system of the present invention is advantageous because it has a special tip adapted for placement into a (pre-treated) root channel to enable filling of the channel from the bottom to the top thereby allowing complete filling of the channel without entrapment of air in the channel or cement. Therefore, the extension element of the present invention preferably has a thin and elongated front end that is preferably tapered from its back to the front end. The angle of taper preferably corresponds to the taper of the posts.

The delivery system and kit according to the present invention reduce manufacturing cost over prior art delivery systems because a dentist can use a single capsule design for various dental materials and dental treatments and simple use the extension element if the dental treatment requires it. For example, when using the delivery system to apply cement to a root channel prior to implantation of a dental post, the capsule with its cannula can be delivered to the dentist together with the extension element. The extension element is easily connectable to the cannula by the dentist to establish the "user-activatable, unreleasable engagement". If the treatment or dental material does not require a long tip, the same capsule design can be used without the extension element. Such flexible delivery system is very convenient from a dental material manufacturer's point of view because manufacture of a single capsule design for various applications will permit reduced costs for manufacture of the capsule, for filling the capsules and for packaging and delivery of the capsules to the dentist.

### Brief description of the drawings

The present invention will be described in more detail in the following with reference to the drawings in which:
- Fig. 1: shows an exemplary capsule with cannula of the delivery system according to the present invention;
- Fig. 2: shows a perspective view of the cannula of the capsule of the delivery system according to the present invention;
- Fig. 3a: shows a perspective view of an exemplary extension element of the delivery system according to Fig. 1;
- Fig. 3b: shows a side view of the extension element of Fig. 3a;
- Fig. 3c: shows a cross-sectional view of the extension element of Fig. 3a along line A-A;
- Fig. 4: shows a perspective view of a modified extension element as shown in Fig. 3a;
- Fig. 5: shows a further modified extension element shown is Fig. 3a;
- Fig. 6: shows schematically the user activation of the delivery system of the example of Figs. 2 to 5;
- Fig. 7: shows an extension element of a delivery system according to the present invention;
- Fig. 8: shows the capsule of Fig. 1 and the extension element of Fig. 3a prior to their engagement with each other; and
- Fig. 9: shows the extension element of Fig. 3a locked to the capsule of Fig. 1.

### Detailed description of the preferred embodiments

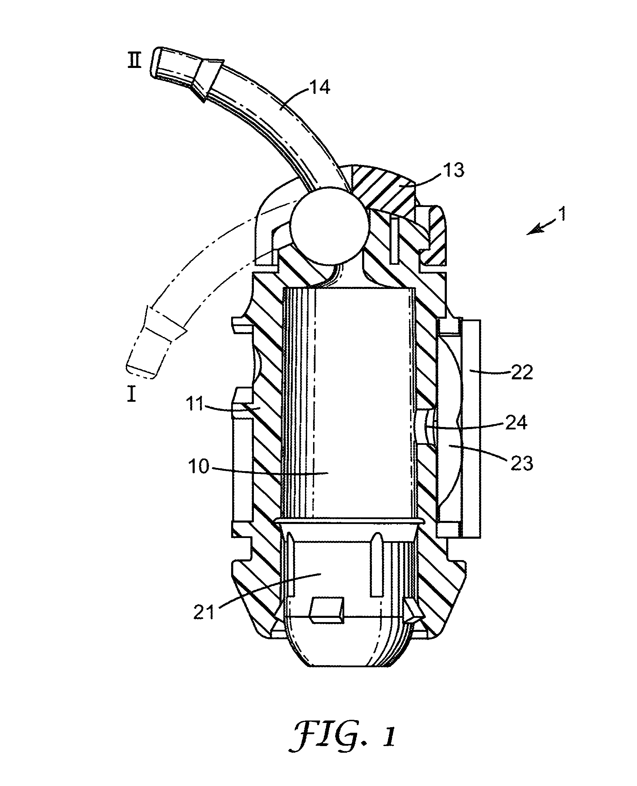

The capsule 1 shown in FIG. 1 comprises a capsule body member 11 having a chamber 10 containing a first dental material component, a piston 21 being arranged within the chamber and adapted to be moved forward within the chamber 10 in direction of the longitudinal axis of the capsule body, a pivot cannula 14 adapted to be swivelled between a rest position I and an operative position II, a cap 13 providing fixture but allowing swivelling of the pivot cannula at the capsule body member, a foil bag 23 arranged at the outer wall of the capsule body containing a second dental material component, and a clip 22 holding the mentioned foil bag 23.

Details relating to the capsule 1, structure and mounting of the clip and the foil bag are described in US-A-3 907 106 and EP-0 157 121, which disclosure is hereby incorporated by reference.

When the capsule 1 shown in FIG. 1 is delivered from the manufacturer to the user, it will be charged with the two components of a dental composition, one being typically a powder contained in the chamber 10 and the other being typically a liquid contained in the foil bag 23. The cannula 14 will be in the rest position I in which position no fluid communication between the cannula 14 and the chamber 10 is provided.

To mix the components, the clip 22 is first depressed in a direction perpendicular to the longitudinal axis of the capsule body so as to break the foil bag 23 and transfer the liquid material through the hole 24 into the mixing compartment 10. Then the capsule 1 is placed into a shaker and is shaken for the desired period of time.

When the capsule 1 is removed from the shaker, the dental composition (for instance a paste) should be ready for use. Thereupon, the cannula 14 may be pivoted to the operative position II, thereby providing fluid communication between the cannula 14 and the chamber 10 and allowing the composition to be displaced from the chamber 10 through the cannula 14 by moving the piston 21 forward, usually by means of a dispensing tool like those known readily known to one skilled in the art (not shown).

Alternatively, instead of the previously described capsule for powder/liquid components capsules (or syringes) with only one or with two or more compartments for storing dental material components (for instance liquid and/or pasty components) can be used in the present invention.

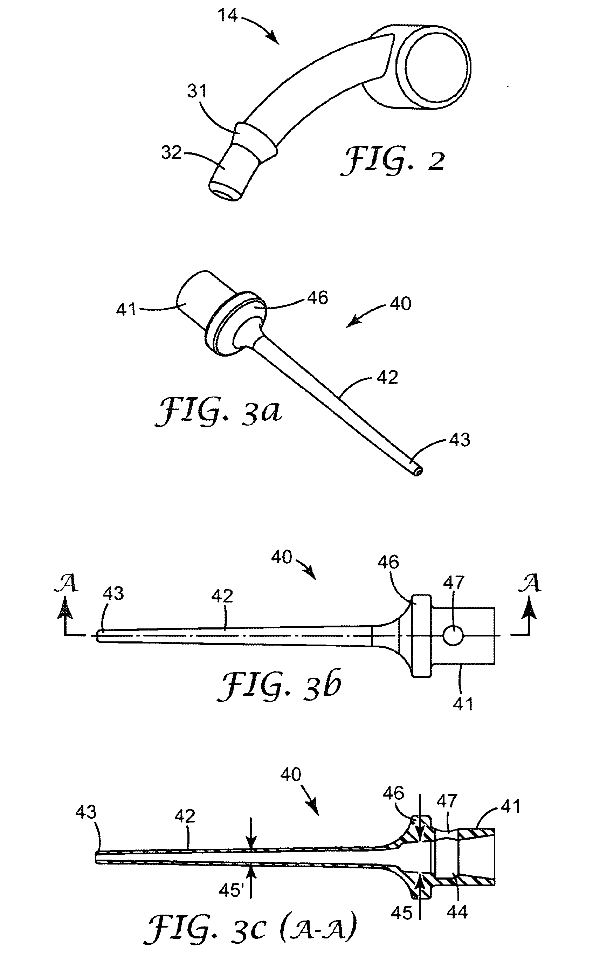

Fig. 2 shows in more detail a pivotable cannula 14 of a capsule 1 of a delivery system of the present invention. As shown in Fig. 2, the cannula 14 may be curved. According to the example shown in Fig. 2, the cannula 14 comprises at least one circumferential conical rim 31 located at a front end area 32 of the cannula 14. Alternatively, one or more retention teeth could be arranged at the circumference of the cannula instead of the conical rim 31.

The extension element 40, 50, and 60, shown in Figs. 3a-3c, 4, 5, and 6, is unreleasably engageable with cannula 14 shown in Figs. 1 and 2.

The extension element 40 shown in Figs. 3a to 3c comprises a tubular back end portion 41 and a tip portion 42. A flange 46 is provided at the transition between the tubular back end portion 41 and the tip portion 42. As can clearly be seen in Figs. 3a to 3c, the average outer diameter of the tubular back end portion 41 is larger than the average outer diameter of the tip portion 42. Furthermore, tip portion 42 is tapered towards its front end 43.

The interior configuration of the extension element 40 is shown in Fig. 3c. The back end portion 41 comprises a first internally conically shaped portion at the back end. That is, the inner diameter of the extension element 40 decreases from the back end in the direction away from the back end, i.e. towards the tip. The truncated cone portion is followed by the mating recess or retention groove, respectively, 44. Preferably, the inner diameter of the extension element 40 at the retention groove 44 corresponds to the largest diameter of the first truncated cone portion. That is, the diameter decreases along the truncated cone and then steps back to the initial diameter so that the decrease in diameter at the cone forms a constriction of the initial inner diameter of the back end portion 41 of the extension element 40. Next to the retention groove 44, a second, preferably conically shaped, portion is present, which is then followed by the tip portion 42. The second portion accommodates the tip portion or front end area, respectively, 32 of the cannula 14 that extends beyond the rim 31 (see Fig. 2). The average diameter 45 of the second portion is larger than the average inner diameter 45' of the tip portion. Inner diameter 45 preferably corresponds to the outer diameter of the front end 32 of cannula 14 so as to provide a tight fit when the extension element 40 is connected to the cannula 14. As an option, the inner diameter 45 can be tapered in the direction towards the tip portion from a diameter substantially corresponding to the outer diameter of the front end 32 of the cannula 14 to a slightly smaller diameter so as to provide better sealing between extension element 40 and cannula 14 when they are fitted together.

Fig. 3c also shows the step in diameter between the retention groove 44 and the first conical portion of the tubular back end portion 41. In such configuration, the extension element 40 can be unreleasably snapped onto the rim 31 of the cannula 14. The retention groove 44 and the annular rim 31 can form a secure interlock when the extension element 40 is connected to the cannula 14.

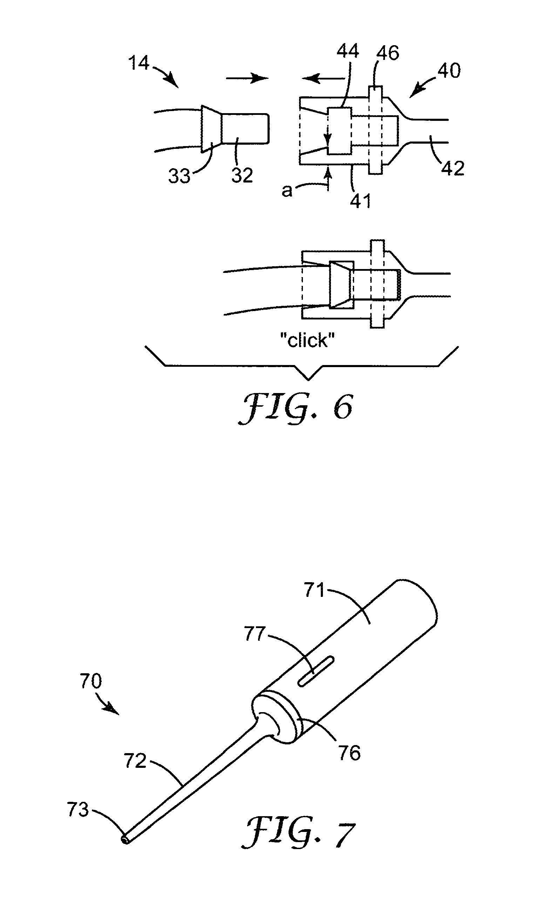

The user activated, unreleasable engagement between the extension element 40 and the cannula 14 is more clearly shown in Fig. 6. In the upper drawing of Fig. 6, the two arrows indicate how the extension element 40 and the cannula 14 can be brought into contact with each other until the tubular back end portion 41 is snapped onto the cannula 14 (see lower drawing in Fig. 6). The back end edge of the retention groove 44 is snapped behind the edge of the rim 31. The wall thickness of the extension element at the back end edge of the retention groove 44, i.e. the wall thickness in the area where the first conically shaped portion has its smallest diameter is identified in Fig. 6 as "a". This wall thickness "a" is preferably between 0.5 and 1.0 mm. In order to allow the tubular back end portion 41 to be easily pushed over the rim 31, this wall thickness "a" is such to leave sufficient elasticity to provide expansion. On the other hand, the wall thickness around the bore is designed to keep sufficient stiffness to provide an unreleasable fit. The force required to snap the extension element 40 onto the cannula 40 preferably is below 100 N. A preferred range of snap on force is less than 80 N and more than 40 N.

Preferably, the snap of the extension element 40 onto the cannula 14 causes an audible "click" sound to provide feedback to the dentist about when the extension element 40 is properly connected to the cannula 14.

Preferably, the cannula 14 is made from a stiff material such as polycarbonate, and the extension element 40 is made from a more elastic material like polypropylene or polyethylene.

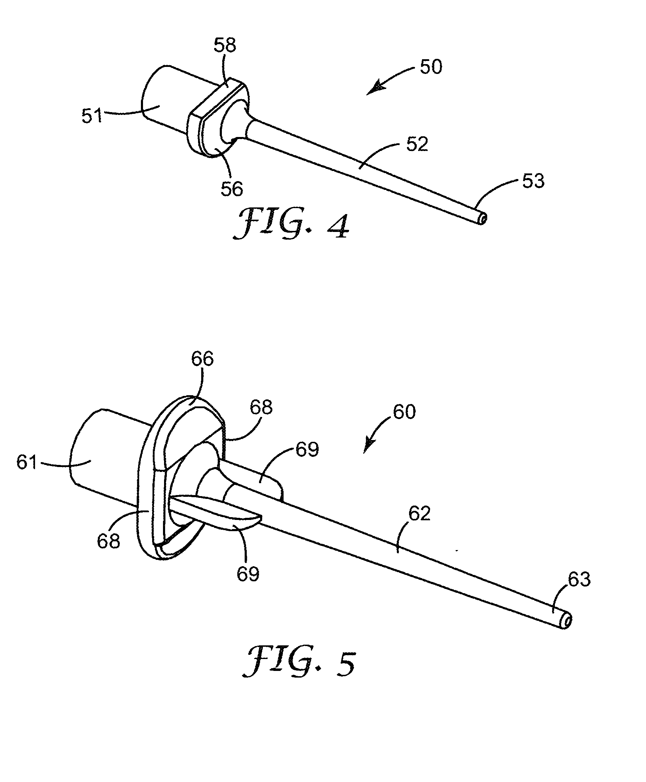

A modified design of the extension element is shown in Fig. 4. Like the extension element 40 shown in Figs. 3a to 3c, extension element 50 comprises a back end portion 51, a tip portion 52 with front end 53, and a flange 56. However, as shown in Fig. 4, flange 56 comprises a flattened portion 58.

Fig. 5 shows a further modified design of the extension element. Extension element 60 comprises a back end portion 61, tip portion 62 with front end 63, and flange 66. Flange 66 comprises two opposite flattened portions 68. Furthermore, two substantially opposite wings or webs 69, respectively, are provided. The wings or webs 69 extend radially from said tip portion 62, and extend along the longitudinal axis of the extension element 60 from the vicinity of the flange 66 towards the front end 63 of the tip portion 62. It is preferred that the webs or wings 69 do not extend along the entire length of the tip portion 62, as shown in Fig. 5.

Fig. 7 shows the embodiment of the present invention. According to this embodiment, the extension element 70 comprises a tubular back end portion 71 that is elastic. In this embodiment, the tip portion 72 is preferably made from a more rigid material than the tubular back end portion 71. Such an extension element 70 is preferably manufactured by a two-shot injection molding process comprising the steps of first molding the tip portion 72 using a more rigid material like polypropylene and a second step of molding the back end portion 71 directly onto a part of the surface of the tip portion using a material like a thermoplastic elastomer (e.g. TPU, SEBS, silicone, rubber etc.).

The extension element 70 according to the present invention preferably does not comprise a retention groove like the example. Proper unreleasable fixation of the extension element at the cannula 14 is provided by friction. Once the extension element is placed with its elastic tubular back end portion 71 on the cannula 14, it is hard to pull it off again because pulling the extension element 70 causes the elastic portion(s) to constrict, thereby increasing the frictional resistance between the outer surface of the cannula and the inner surface of the elastic tubular back end portion 71. This embodiment may also be used with a cannula having no retention rim 31 as the fixation is provided by friction only.

As an option, the extension element 70 of the present invention may also comprise a retention groove.

Furthermore, as also shown in Fig. 7, the extension element 70 comprises an aperture 77 which prevents the impairment of the frictional seal of the extension element 70 on the cannula 14 by allowing any dental material that follows between the inner surface of the elastic tubular end portion and the outer surface of the cannula 14 to escape through the aperture 77.

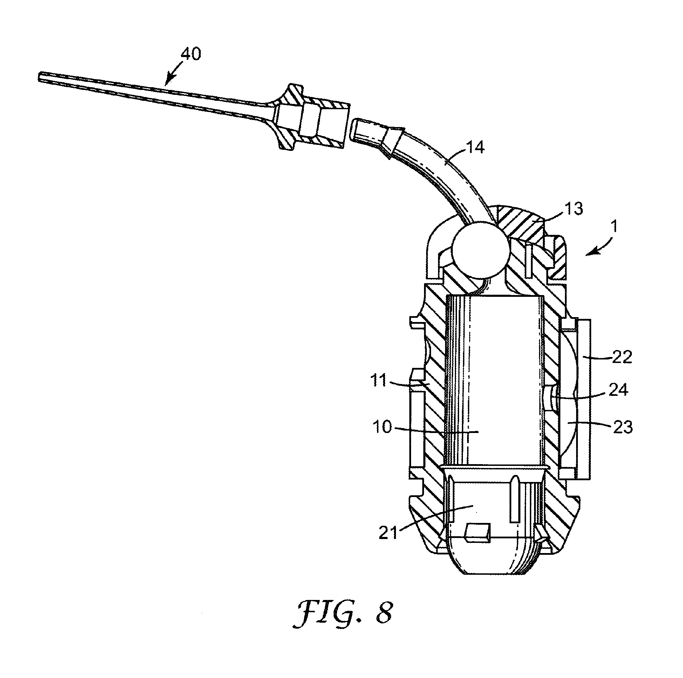

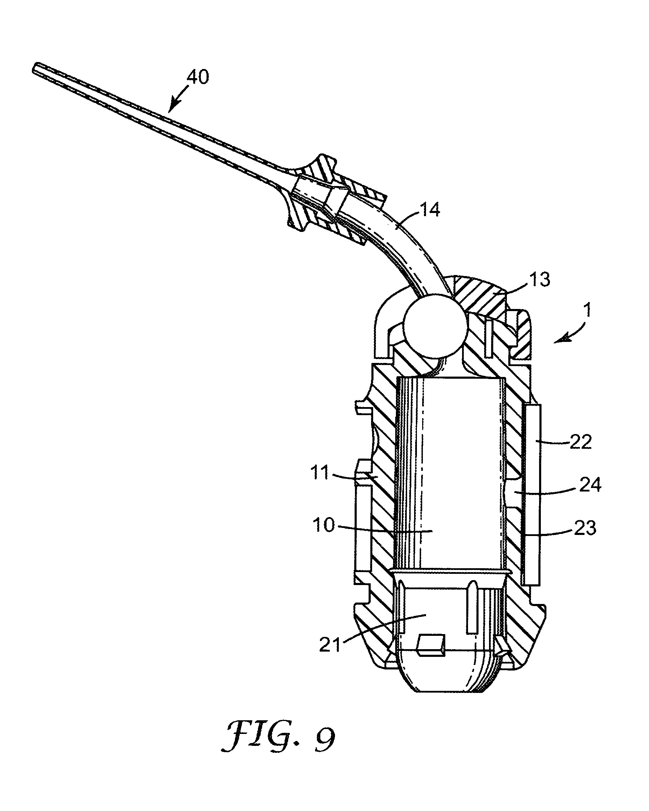

Fig. 8 shows the capsule 1 of Fig. 1 and the cannula 14 and extension element 40 of Figs. 2 and 3 immediately prior to locking the extension element 40 to the cannula 14 of the capsule 1. The engaged state is shown in Fig. 9. Fig. 9 shows how the rim 31 of the cannula 14 has snapped into the groove 44 of the extension element 40. Mixed dental material contained in the chamber 10 can now be dispensed through the cannula 14 and the extension element 40 using the piston 21. A comparison of Figs. 8 and 9 shows that in Fig. 9, foil bag 23 is now emptied through hole 24.

## Claims

1. Delivery system for dental materials comprising:
- a capsule (1) having a capsule body member (11) and a cannula (14) for dispensing material from said capsule body member; and
- an extension element (70);
- wherein said extension element (70) comprises a tubular back end portion (71) having a a first average inner diameter, and a tip portion (72) having a second average inner diameter, said second average inner diameter being smaller than said first average inner diameter,
- **characterized in that** said cannula (14) and said extension element (70) are adapted for a user-activatable unreleasable engagement with each other, and
- wherein said tubular back end portion (71) of said extension element (70) is made from an elastic material, and
- wherein the elasticity of said tubular back end portion (71) is such that pulling the tubular back end portion (71) causes stretching and constriction of the elastic portions of the tubular back end portion (71) which result in increased frictional resistance between the cannula (14) and the extension element (70).

2. The delivery system of claim 1, wherein said tip portion (72) is tapered towards its from end (73).

3. The delivery system of claim 1 or 2, wherein the inner shape of said extension element (70) corresponds to the outer shape of said cannula (14) so as to provide a tight fit.

4. The delivery system of claim 1, wherein said extension element (70) comprises a at least one aperture (77) extending from the interior of the tubular back end portion (71) to its outer surface.

5. The delivery system of any of the preceding claims, wherein said capsule (1) comprises a first chamber for a first dental component, and a second chamber for a second dental component, and wherein said capsule (1) is activatable so as to provide for mixing of said first and second dental materials in said first chamber.

6. The delivery system of claim 5, containing glass ionomer cement, resin modified glass ionomer cements, or a self-adhesive resin modified glass ionomer cement.

7. Kit for delivery of dental materials comprising:
a delivery system according to claim 1 and
an orally implantable post.

8. Kit for delivery of dental materials of claim 7 additionally comprising dental posts.

## Patentansprüche

1. System zur Zuführung dentaler Werkstoffe, das Folgendes aufweist:
- eine Kapsel (1) mit einem Kapselkörperglied (11) und einer Kanüle (14) zum Abgeben von Werkstoff aus dem Kapselkörperglied (11); und
- ein Erweiterungselement (70);
- wobei das Erweiterungselement (70) einen rohrförmigen Hinterendenabschnitt (71) mit einem ersten durchschnittlichen Innendurchmesser und einen Spitzenabschnitt (72) mit einem zweiten durchschnittlichen Innendurchmesser aufweist, wobei der zweite durchschnittliche Innendurchmesser kleiner als der erste durchschnittliche Innendurchmesser ist;
- **dadurch gekennzeichnet, dass** die Kanüle (14) und das Erweiterungselement (70) dazu ausgelegt sind, miteinander in einem vom Benutzer aktivierbaren, unlösbaren Eingriff zu stehen; und
- wobei der rohrförmige Hinterendenabschnitt (71) von dem Erweiterungselement (70) aus einem elastischen Material hergestellt ist, und
- wobei die Elastizität von dem rohrförmigen Hinterendenabschnitt (71) derart ist, dass ein Ziehen an dem rohrförmigen Hinterendenabschnitt (71) eine Ausdehnung und Einschnürung von den elastischen Abschnitten des rohrförmigen Hinterendenabschnitts (71) verursacht, was einen erhöhten Reibungswiderstand zwischen der Kanüle (14) und dem Erweiterungselement (70) zur Folge hat.

2. Zuführungssystem nach Anspruch 1, wobei der Spitzenabschnitt (72) sich in Richtung auf sein Vorderende (73) verjüngt.

3. Zuführungssystem nach Anspruch 1 oder 2, wobei die innere Form des Erweiterungselements (70) der äußeren Form der Kanüle (14) entspricht, wodurch eine Presspassung bereitgestellt ist.

4. Zuführungssystem nach Anspruch 1, wobei das Erweiterungselement (70) mindestens eine Öffnung (77) aufweist, die sich von dem Inneren des rohrförmigen Hinterendenabschnitts (71) bis an seine Außenfläche erstreckt.

5. Zuführungssystem nach einem der vorhergehenden Ansprüche, wobei die Kapsel (1) eine erste Kammer für eine erste Dentalkomponente und eine zweite Kammer für eine zweite Dentalkomponente aufweist, und wobei die Kapsel (1) aktivierbar ist, um hierdurch ein Mischen von dem ersten und dem zweiten dentalen Werkstoff in der ersten Kammer vorzusehen.

6. Zuführungssystem nach Anspruch 5, das einen Glasionomerzement, harzmodifizierte Glasionomerzemente oder einen selbsthaftenden harzmodifizierten Glasionomerzement enthält.

7. Kit zur Zuführung dentaler Werkstoffe, das Folgendes aufweist:
ein Zuführungssystem nach Anspruch 1 und einen oral implantierbaren Stift.

8. Kit zur Zuführung dentaler Werkstoffe nach Anspruch 7, das zusätzlich Wurzelstifte aufweist.

## Revendications

1. Système de distribution pour des matériaux dentaires comportant :
- une capsule (1) possédant un élément (11) corps de capsule et une canule (14) pour administrer un matériau depuis ledit élément corps de capsule ; et
- un élément (70) prolongateur ;
- dans lequel ledit élément (70) prolongateur comporte une section (71) d'extrémité tubulaire arrière d'un premier diamètre intérieur moyen, et une section (72) de bout d'un second diamètre intérieur moyen, ledit second diamètre intérieur moyen étant plus petit que ledit premier diamètre intérieur moyen,
- **caractérisé en ce que** ladite canule (14) et ledit élément (70) prolongateur sont conçus pour une mise en prise activable par l'utilisateur de manière non libérable l'un avec l'autre, et
- dans lequel ladite section (71) d'extrémité tubulaire arrière dudit élément (70) prolongateur est dans un matériau élastique, et
- dans lequel l'élasticité de ladite section (71) d'extrémité tubulaire arrière est telle que lorsque l'on tire la section (71) d'extrémité tubulaire arrière les sections élastiques de la section (71) d'extrémité tubulaire arrière s'étirent et se rétrécissent, ce qui a pour résultat d'augmenter la résistance de frottement entre la canule (14) et l'élément (70) prolongateur.

2. Système de distribution selon la revendication 1, dans lequel ladite section (72) de bout est amincie vers son extrémité (73) avant.

3. Système de distribution selon la revendication 1 ou 2, dans lequel la forme intérieure dudit élément (70) prolongateur correspond à la forme extérieure de ladite canule (14) de façon à fournir un ajustement serré.

4. Système de distribution selon la revendication 1, dans lequel ledit élément (70) prolongateur comporte au moins une ouverture (77) s'étendant de l'intérieur de la section (71) d'extrémité tubulaire arrière à sa surface extérieure.

5. Système de distribution selon l'une quelconque des revendications précédentes, dans lequel ladite capsule (1) comporte un premier compartiment pour un premier composant dentaire, et un second compartiment pour un second composant dentaire, et dans lequel ladite capsule (1) est activable pour permettre un mélange desdits premier et second matériaux dentaires dans ledit premier compartiment.

6. Système de distribution selon la revendication 5, contenant un ciment de verre ionomère, des ciments de verre ionomère modifiés par de la résine, ou un ciment de verre ionomère auto-adhésif modifié par de la résine.

7. Kit destiné à distribuer des matériaux dentaires comportant :
un système de distribution selon la revendication 1 et un tenon implantable par voie orale.

8. Kit destiné à distribuer des matériaux dentaires selon la revendication 7 comportant en supplément des tenons dentaires.
